(19) Europäisches Patentamt
European Patent Office
Office européen des brevets

(11) Veröffentlichungsnummer: **0 163 609**
**A2**

# (12) EUROPÄISCHE PATENTANMELDUNG

(21) Anmeldenummer: 85810245.2

(22) Anmeldetag: 23.05.85

(51) Int. Cl.⁴: C 08 K 5/34
C 07 D 487/04
//(C07D487/04, 209:00, 209:00)

(30) Priorität: 30.05.84 CH 2665/84

(43) Veröffentlichungstag der Anmeldung:
04.12.85 Patentblatt 85/49

(84) Benannte Vertragsstaaten:
CH DE FR GB IT LI

(71) Anmelder: CIBA-GEIGY AG
Postfach
CH-4002 Basel(CH)

(72) Erfinder: Rochat, Alain Claude, Dr.
Route de Schiffenen 38
CH-1700 Fribourg(CH)

(72) Erfinder: Iqbal, Abul, Dr.
Im Schaiengarten 1
CH-4107 Ettingen(CH)

(72) Erfinder: Pfenninger, Johannes, Dr.
Route du Confin 23
CH-1723 Marly(CH)

(72) Erfinder: Cassar, Luigi, Dr.
Via Europa 42
I-20097 S. Donato Milanese (Milano)(IT)

(54) **Verfahren zum Färben von hochmolekularem organischem Material, neue polycyclische Verbindungen sowie deren Herstellung.**

(57) Verfahren zum Färben von hochmolekularem organischem Material in der Masse, gekemzeichnet durch die Verwendung eines 2, 5-Diketopyrrolo-[2, 3-b]-pyrrols der Formel I

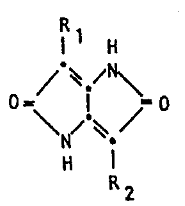

(I)

worin $R_1$ und $R_2$ iscyclische oder heterocyclische aromatische Reste, vorzugsweise gegebenenfalls substituierte Phyenylreste bedeuten.

Die erhaltenen Färbungen, beispielsweise in Kunststoffen, Fasern, Lacken und Drucken, zeichnen sich durch gute Pigmenteigenschaften wie Ueberlackier-, Migrations-, Hitze-, Licht-, und Wetterbeständigkeit.

EP 0 163 609 A2

Croydon Printing Company Ltd

CIBA-GEIGY AG 3-14900/=

Basel (Schweiz)

Verfahren zum Färben von hochmolekularem organischem Material, neue polycyclische Verbindungen sowie deren Herstellung

Die Erfindung betrifft das Färben von hochmolekularem organischem Material mit 2,5-Diketopyrrolo-[2,3-b]-pyrrolen, neue 2,5-Diketo-pyrrolo-[2,3-b]-pyrrole sowie deren Herstellung.

Das 2,5-Diketo-3,6-diphenylpyrrolo-[2,3-b]-pyrrol ist in der Zeitschrift für Naturforschung 32c, S. 292-293 (1977) beschrieben, wobei dieses Produkt im Gemisch mit dem entsprechenden Pulvinsäurelactam erhalten wurde und als rotorange Nadeln charakterisiert wird.

Gegenstand der vorliegenden Erfindung ist ein Verfahren zum Färben von hochmolekularem organischem Material in der Masse, dadurch gekennzeichnet, dass ein 2,5-Diketopyrrolo-[2,3-b]-pyrrol der Formel I

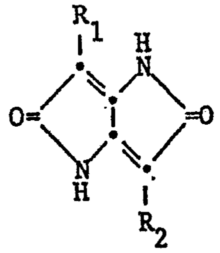

(I)

verwendet wird, wobei $R_1$ und $R_2$ unabhängig voneinander isocyclische oder heterocyclische aromatische Reste bedeuten. Die Formel (I) sowie die nachfolgend aufgeführten Formeln geben nur eine der möglichen tautomeren Strukturen wieder.

Die Reste $R_1$ und $R_2$ können verschieden oder identisch sein.

Bedeuten $R_1$ und $R_2$ isocyclische aromatische Reste, dann vorzugsweise mono- bis tetracyclische, insbesondere mono- oder bicyclische Reste, wie Phenyl, Diphenyl oder Naphthyl, insbesondere aber Phenyl.

Bedeuten $R_1$ und $R_2$ heterocyclische aromatische Reste, dann vorzugsweise mono- bis tricyclische. Diese können rein heterocyclisch sein oder einen heterocyclischen Ring und einen oder mehrere ankondensierte Benzolringe enthalten.
Beispiele von heterocyclischen aromatischen Resten sind Pyridyl, Pyrimidyl, Pyrazinyl, Triazinyl, Furanyl, Pyrrolyl, Thiophenyl, Chinolyl, Cumarinyl, Benzfuranyl, Benzimidazolyl, Benzoxazolyl, Dibenzfuranyl, Benzthiophenyl, Dibenzthiophenyl, Indolyl, Carbazolyl, Pyrazolyl, Imidazolyl, Oxazolyl, Isoxazolyl, Thiazolyl, Indazolyl, Benzthiazolyl, Pyridazinyl, Cinnolyl, Chinazolyl, Chinoxalyl, Phthalazinyl, Phthalazindionyl, Phthalamidyl, Chromonyl, Naphtholactamyl, Chinolonyl, ortho-Sulfobenzoesäureimidyl, Maleinimidyl, Naphtharidinyl, Benzimidazolonyl, Benzoxazolonyl, Benzthiazolonyl, Benzthiazothionyl, Chinazolonyl, Chinoxalonyl, Phthalazonyl, Dioxopyrimidinyl, Pyridonyl, Isochinolonyl, Isochinolinyl, Isothiazolyl, Benzisoxazolyl, Benzisothiazolyl, Indazolonyl, Acridinyl, Acridonyl, Chinazolindionyl, Chinoxalindionyl, Benzoxazindionyl, Benzoxazinonyl und Naphthalimidyl.

Sowohl die isocyclischen wie die heterocyclischen aromatischen Reste können übliche Substituenten aufweisen, wie:

1) Halogenatome, beispielsweise Chlor, Brom, Jod oder Fluor.
2) Verzweigte oder unverzweigte Alkylgruppen mit vorzugsweise 1 bis 18, insbesondere 1 bis 12, vor allem 1 bis 8 und besonders bevorzugt mit 1 bis 4 C-Atomen. Diese Alkylgruppen können weitere nicht-wasserlöslichmachende Substituenten aufweisen, wie beispielsweise Fluor, Hydroxy, Cyano, Alkoxy mit 1 bis 4 C-Atomen oder unsubstituiertes oder durch Halogen, $C_1$-$C_4$-Alkyl oder $C_1$-$C_4$-Alkoxy substituiertes Phenyl. Beispiele von Alkylgruppen sind Methyl, Aethyl, n-Propyl, Isopropyl, n-Butyl, sec.-Butyl, tert.-Butyl, tert.-Amyl, n-Pentyl, n-Hexyl, 1,1,3,3-Tetramethylbutyl, n-Heptyl, n-Octyl oder Benzyl.

3) Die Gruppen $-CF_3$, $-CCl_3$, $-NO_2$ oder -CN.

4) Die Gruppe $-OR_3$, worin $R_3$ Wasserstoff, Alkyl, Aryl, beispielsweise Naphthyl, oder insbesondere unsubstituiertes oder durch Halogen, Alkyl oder Alkoxy substituiertes Phenyl, $C_5$-$C_6$-Cycloalkyl, Aralkyl oder einen heterocyclischen Rest bedeutet. In den Definitionen von $R_3$ vorkommendes Alkyl kann z.B. ein der unter 2) als bevorzugt angegebenen Anzahl C-Atome haben. Als Beispiele von $R_3$ seien genannt: Methyl, Aethyl, n-Propyl, Isopropyl, Phenyl, o-, m- oder p-Chlorphenyl, o-, m- oder p-Methylphenyl, α- oder β-Naphthyl, Cyclohexyl, Benzyl, Thienyl oder Pyranylmethyl.

5) Die Gruppe $-SR_3$, worin $R_3$ die unter 4) angegebene Bedeutung hat. Als Beispiele für $R_3$ seien genannt: Methyl, Aethyl, n-Propyl, Isopropyl, Phenyl, o-, m- oder p-Chlorphenyl, o-, m- oder p-Methylphenyl, α- oder β-Naphthyl, Cyclohexyl, Benzyl, Thienyl oder Pyranylmethyl.

6) Die Gruppe der Formel $-N(R_4)(R_5)$, worin $R_4$ und $R_5$ unabhängig voneinander -H, unsubstituiertes oder durch Cyano oder Hydroxy substituiertes $C_1$-$C_4$-Alkyl, $C_5$-$C_6$-Cycloalkyl, unsubstituiertes oder durch Halogen, $C_1$-$C_4$-Alkyl oder $C_1$-$C_4$-Alkoxy substituiertes Phenyl bedeuten, oder worin $R_4$ und $R_5$ zusammen mit dem N-Atom einen 5 bis 6-gliedrigen heterocyclischen Ring bilden.

Als Beispiele seien genannt: Amino, Methylamino, Dimethylamino,

Aethylamino, Diäthylamino, Isopropylamino, β-Hydroxyäthylamino, β-Hydroxypropylamino, N,N-Bis-(β-hydroxyäthyl)-amino, N,N-Bis-(β-cyanäthyl)-amino, Cyclohexylamino, Phenylamino, N-p-Methylphenylamino, Piperidyl oder Morpholyl.

7) Die Gruppe der Formel $-COOR_3$, worin $R_3$ die unter 4) angegebene Bedeutung hat. Als Beispiele für $R_3$ seien genannt: Methyl, Aethyl, Isopropyl, tert.-Butyl, n-Butyl, Phenyl, Benzyl oder Furfuryl.

8) Die Gruppe der Formel $-COR_3$, worin $R_3$ die unter 4) angegebene Bedeutung hat. Als Beispiele seien genannt: Methyl, Aethyl, tert.-Butyl, Phenyl, o-, m- oder p-Chlorphenyl, o-, m- oder p-Methylphenyl oder α- bzw. β-Naphthyl.

9) Die Gruppen der Formel $-SO_2R_3$ oder $-SOR_3$, worin $R_3$ die unter 4) angegebene Bedeutung hat. Als Beispiele seien genannt: Methylsulfonyl, Aethylsulfonyl, Phenylsulfonyl, 2-Naphthylsulfonyl, Phenylsulfoxid.

10) Die Gruppe der Formel $-CON(R_4)(R_5)$, worin $R_4$ und $R_5$ die unter 6) angegebene Bedeutung haben. Als Beispiele seien genannt: Carbamoyl, N-Methylcarbamoyl, N-Aethylcarbamoyl, N-Phenylcarbamoyl, N,N-Dimethylcarbamoyl, N-Methyl-N-Phenylcarbamoyl, oder N-Piperidylcarbamoyl.

11) Die Gruppe der Formel $-SO_2N(R_4)(R_5)$, worin $R_4$ und $R_5$ die unter 6) angegebene Bedeutung haben. Als Beispiele seien genannt: Sulfamoyl, N-Methylsulfamoyl, N-Aethylsulfamoyl, N-Phenylsulfamoyl, N-Methyl-N-phenylsulfamoyl oder N-Morpholylsulfamoyl.

12) Die Gruppen Methylendioxy, Aethylendioxy, Methylendithio oder Aethylendithio ($-SCH_2CH_2S-$), wobei diese Gruppen an zwei benachbarten Kohlenstoffatomen eines Phenylrings sitzen. Beispiele hierfür sind 2,3-Dihydro-1,4-dioxo-naphthyl oder 2,3-dihydro-1,4-dithio-naphthyl.

Von besonderem Interesse ist die Verwendung der symmetrischen Verbindungen der Formel I, worin $R_1$ und $R_2$ einen Rest der Formel II

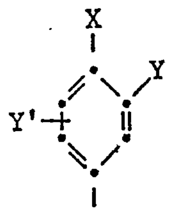

(II)

bedeuten, worin X, Y und Y' unabhängig voneinander H- oder Halogenatome, Carbamoyl-, Nitro-, Cyan-, Trifluormethyl-, Alkyl-, Alkoxy- oder Alkylmercaptogruppen mit 1-4 C-Atomen,Alkoxycarbonyl- oder N-Alkylcarbamoylgruppen mit 2-5 C-Atomen, unsubstituierte oder durch Halogen, Alkyl oder Alkoxy mit 1-4 C-Atomen substituierte Phenoxy-, Phenylmercapto-, Phenoxycarbonyl- oder N-Phenylcarbamoylgruppen bedeuten, wobei mindestens einer der Substituenten X, Y und Y' ein Wasserstoffatom bedeutet.

Bevorzugt sind symmetrische Verbindungen der Formel I, worin $R_1$ und $R_2$ Reste der Formel III

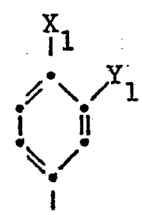

(III)

bedeuten, wobei einer der Substituenten $X_1$ und $Y_1$ ein H-, Chlor- oder Bromatom, eine Methyl- oder Cyangruppe, eine Methoxygruppe, eine Alkoxycarbonyl- oder N-Alkylcarbamoylgruppe mit 2-5 C-Atomen oder eine unsubstituierte oder durch Chlor, Methyl oder Methoxy substituierte Phenoxy-, Phenylmercapto- oder N-Phenylcarbamoylgruppe und der andere ein H-Atom bedeutet.

Die Herstellung der Verbindung der Formel I, worin $R_1$ und $R_2$ Phenyl bedeuten, ist in der Zeitschrift für Naturforschung 32c, 292-293 (1977) durch Reaktion des Pulvinsäuremethylesters (auch Vulpinsäure genannt) der Formel IV mit 2-Hydroxyaethoxyacetamid der Formel V

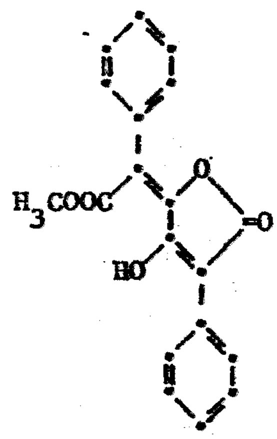

(IV)

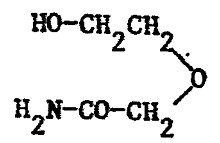

(V)

beschrieben, wobei neben der Verbindung der Formel I (Dilactam der Pulvinsäure, $R_1$, $R_2$ = Phenyl) auch das entsprechende Monolactam der nachstehenden Formel erhalten wurde:

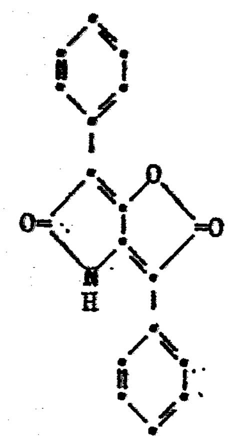

Die übrigen Verbindungen der Formel I sind neu und können beispielsweise ausgehend von substituierten Pulvinsäureestern auf analoge Weise hergestellt werden, wobei anstelle des oben angegebenen 2-Hydroxyaethoxyacetamids auch andere sogenannte Ammoniak-Spender (Ammoniak abgebende Mittel) in Gegenwart eines aliphatischen Alkohols verwendet werden können. Als Ammoniak-Spender kommen beispielsweise Ammoniumsalze wie Ammoniumformiat oder -acetat, ferner aliphatische Amide wie Acetamid, Chloracetamid oder Aethoxyacetamid in Frage.

Die Substituenten in den Resten $R_1$ und $R_2$ der Formel I können auch nachträglich eingeführt oder durch Umwandlung anderer Substituenten erhalten werden.

Die Verbindungen der Formel I erhält man beispielsweise durch Umsetzung eines Pulvinsäurederivates der Formel IX oder eines Pulvinsäurelactons der Formel VI in einem organischen Lösungsmittel in Gegenwart eines Ammoniak abgebenden Mittels und eines aliphatischen Alkohols bei erhöhter Temperatur, vorzugsweise unter Druck

(IX) 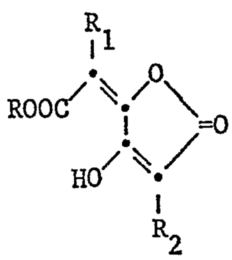 , 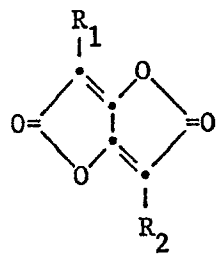 (VI),

wobei $R_1$ und $R_2$ die oben angegebene Bedeutung haben und R für $C_1$-$C_8$-Alkyl steht. Man kann aber auch 2 Derivate der Formel IX bzw. VI mit verschiedenen Substituenten $R_1$ und $R_2$ gleichzeitig zu den Dilactamen der Formel I umsetzen, wobei man Mischkristalle erhalten kann.

Beispiele für Ammoniak abgebende Mittel stellen die auf Seite 6 angegebenen Mittel dar. Als aliphatischen Alkohol verwendet man insbesonders einen Alkohol mit 1 bis 8 C-Atomen oder Gemische davon.

Man führt die Reaktion in einem organischen Lösungsmittel durch. Als Lösungsmittel seien genannt: primäre, sekundäre oder tertiäre Alkohole, Glykole, Aether, Glykolaether, Nitrile, aliphatische oder aromatische Kohlenwasserstoffe, Formamide, Acetamide, N-Methylpyrrolidon oder Nitrobenzol.

Bevorzugt verwendet man einen Alkohol als Lösungsmittel, der gleichzeitig als Reaktionspartner für die Lactonspaltung eingesetzt wird. Die Reaktion wird insbesondere bei einer Temperatur von 120 bis 200°C, vorzugsweise 140 bis 180°C, durchgeführt.

Die benötigten Zwischenprodukte der Formeln VI und IX erhält man z.B. durch Reaktion von zwei Mol einer Verbindung der Formeln $R_1-CH_2CN$ oder $R_2-CH_2-CN$ oder von 1 Mol einer Verbindung der Formel $R_1-CH_2-CN$ und 1 Mol einer Verbindung der Formel $R_2-CH_2-CN$ mit einem Oxalsäurediester zu einem Zwischenprodukt der Formeln VIIa bzw. VIIb oder VIII

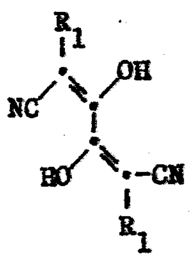

(VIIa)

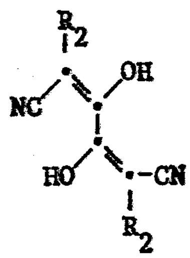

(VIIb)

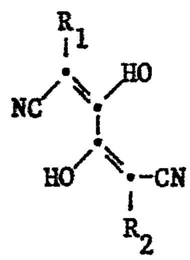

(VIII)

Letztere können dann ihrerseits auf übliche Art und Weise zu Pulvinsäurelactonen der Formel VI oder zu Pulvinsäurederivaten der Formel IX umgesetzt werden.

In den Formeln VI bis IX haben $R_1$, $R_2$ und R die oben angegebene Bedeutung.

Als Oxalsäurediester verwendet man insbesondere die Alkylester mit 1 bis 8 C-Atomen.

Die Herstellung der benötigten Pulvinsäuren-, Pulvinsäureester- und Pulvinsäurelactonderivate ist beispielsweise in US-PS 3 676 464 und US-PS 3 944 571 beschrieben.

Einen weiteren Erfindungsgegenstand bilden die Verbindungen der Formel X

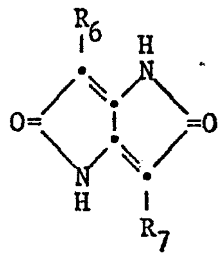

(X),

worin mindestens einer der Reste $R_6$ und $R_7$ einen substituierten Phenylrest bedeutet, ferner $R_6$ und $R_7$ einen carbocyclischen aromatischen oder heterocyclischen aromatischen Rest bedeuten. Die Reste $R_6$ und $R_7$ können gleich oder verschieden sein.

Bevorzugt sind jene Verbindungen der Formel X, worin $R_6$ und $R_7$ identisch sind, insbesondere jene worin beide Reste $R_6$ und $R_7$ substituierte Phenylreste bedeuten.

Bevorzugt sind jene symmetrischen Verbindungen der Formel X, worin $R_6$ und $R_7$ Reste der Formel XI

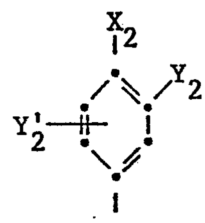

(XI)

bedeuten, worin $X_2$, $Y_2$ und $Y'_2$ unabhängig voneinander Wasserstoff, Halogenatome, Carbamoyl-, Cyan-, Nitro-, Trifluormethyl-, Alkyl-, Alkoxy- oder Alkylmercaptogruppen mit 1-4 C-Atomen, Alkoxycarbonyl- oder N-Alkylcarbamoylgruppen mit 2-5 C-Atomen, unsubstituierte oder durch Halogen, Alkyl oder Alkoxy mit 1-4 C-Atomen substituierte Phenoxy-, Phenylmercapto-, Phenoxycarbonyl- oder N-Phenylcarbamoylgruppen bedeuten, wobei mindestens einer der Substituenten $X_2$, $Y_2$ und $Y'_2$ ein H-Atom bedeutet.

Von besonderem Interesse sind jene symmetrischen Verbindungen der

Formel X , worin $R_6$ und $R_7$ Reste der Formel XII

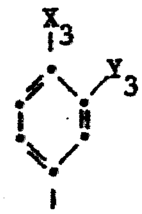

(XII)

bedeuten, worin einer der Substituenten $X_3$ und $Y_3$ ein Chlor- oder Bromatom, eine Methyl- oder Cyangruppe, eine Methoxygruppe, eine Alkoxycarbonyl- oder N-Alkylcarbamoylgruppe mit 2-5 C-Atomen oder eine unsubstituierte oder durch Chlor, Methyl oder Methoxy substituierte Phenoxy-, Phenylmercapto- oder N-Phenylcarbamoylgruppe und der andere ein H-Atom bedeuten.

Bedeuten $R_6$ und $R_7$ Heteroarylreste, dann vorzugsweise mono- bis tricyclische. Diese können rein heterocyclisch sein oder einen heterocyclischen Ring und einen oder mehrere ankondensierte Benzolringe enthalten, welche nicht wasserlöslichmachende Substituenten wie -H, -Cl, $-CH_3$ oder $-OCH_3$ enthalten können.

Als Beispiele für $R_6$ und $R_7$ in der Formel X seien die folgenden Reste genannt:

3- oder 4-Chlorphenyl, 3- oder 4-Bromphenyl, 3- oder 4-Fluorphenyl, 3,4- oder 3,5-Dichlorphenyl, 3- oder 4-Methylphenyl, 3- oder 4-Aethylphenyl, 3- oder 4-Isopropylphenyl, 3,4- oder 3,5-Dimethylphenyl, 3-Chlor-4-methylphenyl, 4-Chlor-3-methylphenyl, 3- oder 4-Methoxyphenyl, 4-Aethoxyphenyl, 3,4-Dimethoxyphenyl, 3- oder 4-Aethoxyphenyl, 3-Methyl-4-methoxyphenyl, 4-Methyl-3-methoxyphenyl, 3-Chlor-4-methoxyphenyl, 4-Chlor-3-methoxyphenyl, 3- oder 4-Phenoxyphenyl, 3- oder 4-o-Chlorphenoxyphenyl, 3- oder 4-o-, m- oder p-Methylphenoxyphenyl, 3- oder 4-Methylmercaptophenyl, 3- oder 4-Phenylmercaptophenyl, 3- oder 4-Nitrophenyl, 3- oder 4-Trifluormethylphenyl, 3,5-Bis-trifluormethylphenyl, 4-Dimethylaminophenyl, 4-Diaethylaminophenyl, 3- oder 4-Methoxy-

carbonylphenyl, 3- oder 4-Aethoxycarbonylphenyl, 3- oder 4-Phenoxycarbonylphenyl, 3- oder 4-Acetylphenyl, 3- oder 4-Benzoylphenyl, 3- oder 4-Carbamoylphenyl, 3- oder 4-N-Methylcarbamoylphenyl, 3- oder 4-Carbamoylphenyl, 3- oder 4-N-Methylcarbamoylphenyl, 3- oder 4-N-Aethylcarbamoylphenyl, 3- oder 4-N-Phenylcarbamoylphenyl, 3- oder 4-Sulfamoylphenyl, 3- oder 4-N-Methylsulfamoylphenyl, 3- oder 4-N-Phenylsulfamoylphenyl, 3- oder 4-Methylsulfonylphenyl, 3- oder 4-Phenylsulfonylphenyl, 4-Diphenylyl, 2-Naphthyl, 9-Phenanthryl, 3-Pyrenyl, 2-Anthrachinonyl, 2-Furanyl, 2-Thienyl, 2-Imidazolyl, 1,3,4-Triazolyl-2, 1,2,4-Triazolyl-3, 5-Tetrazolyl, 2-Oxazolyl, 2-Thiazolyl, 2-Imidazolyl, 2-(1',3',4'-Oxadiazolyl) 5-(1',2',4'-Oxadiazolyl), 6-Benzfuranyl, 3,4-Methylendioxyphenyl, 2-Indolyl, 4-Diphenylenoxidyl, 3-N-Aethylcarbazolyl, 2- oder 5-Benzoxazolyl, 2- oder 5-Benzthiazolyl, 2- oder 5-Benzimidazolyl, 5-Phthalimidyl, 3-Indazolyl, 2-,3- oder 4-Pyridyl, 2-, 3-, 4- oder 6-Chinolyl, 2-, 3- oder 4-Pyrimidyl, Chinolon-2-yl-3, 2-, 4- oder 6-Chinazolyl, 2-Chinoxalyl, 2-Triazinyl-1,3,5, 7-Phenmorpholyl, Naphthostyryl oder 3-Cumarinyl.

Gemäss dem vorliegenden Verfahren können die Verbindungen der Formel I je nach Art ihrer Substituenten und des zu färbenden Polymeren als polymerlösliche Farbstoffe oder insbesondere als Pigmente verwendet werden. Im letzteren Falle ist es vorteilhaft, die bei der Synthese anfallenden Produkte in eine feindisperse Form überzuführen. Dies kann auf verschiedene Weise geschehen, beispielsweise:

a) durch Mahlen oder Kneten, zweckmässig in Gegenwart von Mahlhilfsmitteln, wie anorganischen oder organischen Salzen mit oder ohne Zusatz organischer Lösungsmittel. Nach dem Mahlen werden die Hilfsmittel wie üblich entfernt, lösliche anorganische Salze z.B. mit Wasser und wasserunlösliche organische Lösungsmittel beispielsweise durch Wasserdampfdestillation,

b) durch Umfällen aus Schwefelsäure, Methansulfonsäure, Trichloressigsäure oder Polyphosphorsäure,

c) Ueberführen des Rohpigments in ein Alkali- oder Aminsalz und Hydrolyse des letzteren. Dies geschieht beispielsweise durch Anrühren des Rohpigments mit einer Base, beispielsweise mit einem Alkalihydroxid oder -alkoholat, Ammoniak oder einem Amin in einem polaren organischen Lösungsmittel wie Dimethylformamid, wobei das Pigment ganz oder teilweise in Lösung geht. Durch Hydrolyse, vorzugsweise durch Ansäuern der gegebenenfalls filtrierten Lösung, wird das Pigment ausgefällt.

Es kann sich als zweckmässig erweisen, die nach a), b) oder c) behandelten Pigmente oder die direkt nach ihrer Synthese erhaltenen Produkte mit organischen Lösungsmitteln, vorzugsweise mit solchen, die über 100°C sieden, nachzubehandeln.

Als besonders geeignet erweisen sich durch Halogenatome, Alkyl- oder Nitrogruppen substituierte Benzole, wie Xylole, Chlorbenzol, o-Dichlorbenzol oder Nitrobenzol sowie Pyridinbasen, wie Pyridin, Picolin oder Chinolin, ferner Ketone, wie Cyclohexanon, Aether wie Aethylenglykolmonomethyl- oder -monoäthyläther, Amide wie Dimethylformamid oder N-Methyl-pyrrolidon, sowie Dimethylsulfoxyd, Sulfolan oder Wasser allein, gegebenenfalls unter Druck. Man kann die Nachbehandlung auch in Wasser in Gegenwart von organischen Lösungsmitteln und/oder mit Zusatz von oberflächenaktiven Substanzen oder aliphatischen Aminen, oder im flüssigen Ammoniak durchführen.

Je nach Verwendungszweck erweist es sich als vorteilhaft, die Pigmente als Toner oder in Form von Präparaten zu verwenden.

Das erfindungsgemäss zu färbende hochmolekulare organische Material kann natürlicher oder künstlicher Herkunft sein. Es kann sich z.B. um Naturharze oder trocknende Oele, Kautschuk oder Casein oder um abgewandelte Naturstoffe, wie Chlorkautschuk, ölmodifizierte Alkydharze, Viscose, um Celluloseäther oder Ester, wie Celluloseacetat, Cellulosepropionat, Celluloseacetobutyrat oder Nitrocellulose handeln, insbesondere aber um vollsynthetische organische Polymere (Duroplaste und Thermoplaste), wie sie durch Polymerisation, Polykondensation oder Polyaddition erhalten werden. Aus der Klasse der Polymerisationsharze seien in erster Linie genannt: Polyolefine, wie Polyäthylen, Polypropylen oder Polyisobutylen, ferner substituierte Polyolefine, Polymerisate aus Vinylchlorid, Vinylacetat, Styrol, Acrylnitril, Acrylsäure- und/oder Methacrylsäureestern oder Butadien, sowie Kopolymerisate der erwähnten Monomeren, insbesondere ABS oder EVA.

Aus der Reihe der Polyadditions- und Polykondensationsharze seien die Kondensationsprodukte von Formaldehyd mit Phenolen, die sogenannten Phenoplaste, und die Kondensationsprodukte von Formaldehyd mit Harnstoff, Thioharnstoff und Melamin, die sogenannten Aminoplaste, die als Lackharze verwendeten Polyester, und zwar sowohl gesättigte, wie z.B. Alkydharze, als auch ungesättigte, wie beispielsweise Maleinatharze, ferner die linearen Polyester und Polyamide oder Silikone genannt.

Die erwähnten hochmolekularen Verbindungen können einzeln oder in Gemischen, als plastische Massen oder Schmelzen, die gegebenenfalls zu Fasern versponnen werden können, vorliegen.

Die erwähnten hochmolekularen organischen Materialien können auch in gelöster Form als Filmbildner oder Bindemittel für Lacke oder Druckfarben vorliegen, wie z.B. Leinölfirnis, Nitrocellulose, Alkydharze, Melaminharze und Harnstoff-Formaldehydharze oder Acrylharze.

Die Pigmentierung der hochmolekularen, organischen Substanzen mit den Pigmenten der Formel I erfolgt beispielsweise derart, dass man ein solches Pigment gegebenenfalls in Form von Masterbatches, diesen Substraten unter Verwendung von Walzwerken, Misch- oder Mahlapparaten zumischt. Das pigmentierte Material wird hierauf nach an sich bekannten Verfahren wie Kalandrieren, Pressen, Strangpressen, Verspinnen, Streichen, Giessen oder durch Spritzguss in die gewünschte endgültige Form gebracht. Oft ist es erwünscht, zur Herstellung von nicht starren Formlingen oder zur Verringerung ihrer Sprödigkeit, den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können im erfindungsgemässen Verfahren vor oder nach der Einverleibung des Pigmentfarbstoffes in die Polymeren eingearbeitet werden. Es ist ferner möglich, zwecks Erzielung verschiedener Farbtöne den hochmolekularen, organischen Stoffen neben den Verbindungen der Formel I noch Füllstoffe bzw. andere farbgebende Bestandteile wie Weiss-, Bunt- oder Schwarzpigmente in beliebigen Mengen zuzufügen.

Zum Pigmentieren von Lacken und Druckfarben werden die hochmolekularen organischen Materialien und die Verbindungen der Formel I gegebenenfalls zusammen mit Zusatzstoffen wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Die erhaltenen Färbungen, beispielsweise in Kunststoffen, Fasern, Lacken und Drucken zeichnen sich durch eine gute Dispergierbarkeit, gute Ueberlackier-, Migrations-, Hitze-, Licht- und Wetterbeständigkeit sowie durch eine hohe Farbstärke aus.

In den nachfolgenden Beispielen bedeuten die Teile, sofern nichts anderes angegeben wird, Gewichtsteile und die Prozente Gewichtsprozente.

Beispiel 1: 2,5-Diketo-3,6-diphenyl-pyrrolo-[2,3-b]-pyrrol

a) Herstellung des Rohprodukts

6,51 g Vulpinsäure werden zusammen mit 2,08 g sublimiertem Aethoxyacetamid und 1,25 g Aethylenglykol in ein kleines Druck-Reagenzglas von max. 40 ml Inhalt eingefüllt. Dieses Reagenzglas wird mit einem speziellen Schraubdeckel (inkl. Sicherheitsventil) verschlossen und unter langsamen Drehen in einer schrägen Stellung auf ca. 160°C (Bad-Temperatur) stufenweise geheizt. Ab 150°C ist die ganze Masse durchgeschmolzen, wobei sie sich allmählich dunkel-rot färbt. Man hält das Reagenzglas 14 Stunden bei dieser Temperatur, lässt es anschliessend abkühlen und öffnet es bei Raumtemperatur. Das so erhaltene Reaktionsgemisch wird schliesslich mit 30 ml Aceton versetzt, kräftig verrührt, filtriert, mit Aceton, dann Methanol und schliesslich mit Wasser gewaschen und bei ca. 90-100°C unter Vakuum getrocknet. Man erhält ein gelbes Pulver, das etwa 85 Gew-% 2,5-Diketo-3,6-diphenyl-pyrrolo-[2,3-b]-pyrrol und ca. 15 Gew.-% 2,5-Diketo-3,6-diphenyl-pyrrolo-[2,3-b]-furan enthält.

b) Reinigung des Rohproduktes

500 mg des gemäss 1a) erhaltenen Rohprodukts werden in 8 ml Dimethylformamid angeschlämmt und mit einer Lösung von 0,62 g 30 %-igem Natriummethylat (in Methanol) in 3 ml Dimethylformamid langsam versetzt. Es entsteht eine rote Lösung, die man während ca. 1 Std.

bei Raumtemperatur ausrühren lässt. Anschliessend dosiert man innert 5 Minuten 10 ml destilliertes Wasser langsam hinzu und erwärmt die erhaltene Suspension auf ca. 80°C. Das Gemisch wird filtriert, mit Methanol, dann mit heissem Wasser gewaschen und im Vakuumofen bei 110°C getrocknet. Man erhält 2,5-Diketo-3,6-diphenyl-pyrrolo[2,3-b]-pyrrol der nachstehenden Formel als rotes Pulver in 75 % Ausbeute, bezogen auf das eingesetzte Rohprodukt, das in einer N-Methyl-pyrrolidon-Lösung ein Absorptionsmaximum $\lambda_{max}$ bei 366 nm mit einem $\varepsilon$-Wert von 25800 aufweist.

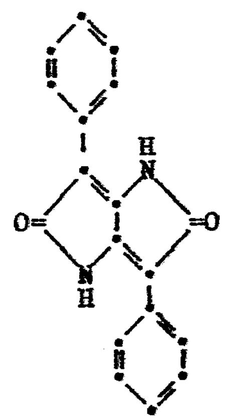

Beispiel 2: 2,5-Diketo-3,6-di-(3',4'-dimethoxyphenyl)-pyrrolo[2,3-b]-pyrrol

a) Herstellung des Rohprodukts

13,34 g 3,3',4,4'-Tetramethoxy-pulvinsäurelacton werden zusammen mit 3,33 g sublimiertem Aethoxyacetamid, 1,30 ml Methanol und 1,80 ml Aethylenglykol in ein kleines Druck-Reagenzglas von max. 80 ml Inhalt eingefüllt. Dieses Reagenzglas wird mit einem speziellen Schraubdeckel (inkl. Sicherheitsventil) verschlossen und unter langsamem Drehen in einer schrägen Stellung auf ca. 160°C (Bad-Temperatur) stufenweise erhitzt. Ab etwa 150°C ist die ganze Masse durchgeschmolzen, wobei sie sich allmählich dunkel-rot färbt. Man hält das Reagenzglas 16 Stunden bei dieser Temperatur, lässt es anschliessend abkühlen und öffnet es bei Raumtemperatur. Das erhaltene Reaktionsgemisch wird mit verschiedenen Portionen von Lösungsmitteln

aus dem Reagenzglas herausgespült (2 Mal mit 50 ml Methanol dann 2 Mal mit 50 ml Aceton). Alle Fraktionen werden vereinigt, auf Rückfluss gekocht und filtriert. Das so isolierte unlösliche Produkt wird mit Aceton, dann Methanol und schliesslich mit Wasser gewaschen und im Vakuumschrank bei 90-100°C getrocknet. Man erhält ein rotes Pulver, das etwa 80 Gew.-% 2,5-Diketo-3,6-di-(3',4'-dimethoxyphenyl)-pyrrolo-[2,3-b]-pyrrol und etwa 20 Gew.-% 2,5-Diketo-3,6-di-(3',4'-dimethoxyphenyl)-pyrrolo-[2,3-b]-furan enthält.

b) Reinigung des Rohprodukts

938 mg des gemäss Bspl. 2a) erhaltenen Rohprodukts werden in 20 ml Dimethylformamid angeschlämmt und mit einer Lösung von 0,84 g 30 %-igem Natriummethylat (in Methanol) in 4 ml Dimethylformamid langsam versetzt. Es entsteht eine rote Lösung, die man während ca. 1 Std. bei Raumtemperatur ausrühren lässt. Anschliessend dosiert man in ca. 30 Minuten 40 ml destilliertes Wasser hinzu und erwärmt die erhaltene Suspension auf ca. 60°C. Nach 1-stündigem Stehenlassen bei dieser Temperatur wird das erhaltene Gemisch filtriert, mit Methanol, dann mit heissem Wasser gewaschen und im Vakuumofen bei 100°C getrocknet. Man erhält 2,5-Diketo-3,6-di-(3',4'-dimethoxyphenyl)-pyrrolo-[2,3-b]-pyrrol der nachstehenden Formel als rotes Pulver, das in einer Dimethylformamid-Lösung ein Absorptionsmaximum $\lambda_{max}$ bei 400 nm mit einem $\varepsilon$-Wert von 23400 aufweist und PVC-Folien in gelbem Farbton färbt.

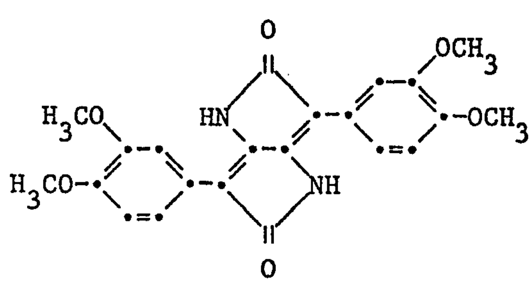

Beispiele 3 bis 6: In der nachstehenden Tabelle I werden weitere Verbindungen der Formel I aufgeführt, welche analog dem Beispiel 2 und ausgehend vom entsprechenden Pulvinsäurelacton hergestellt werden.

Tabelle I

| Beispiel Nr. | Erhaltene Verbindung der Formel I | | $\lambda_{max}$ | $\varepsilon_{max}$ |
|---|---|---|---|---|
| | $R_1$ | $R_2$ | (in DMFA*) | |
| 3 | 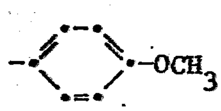 | 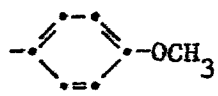 | 358 nm | 22000 |
| 4 | 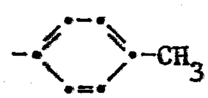 | 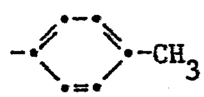 | 370 nm | 26200 |
| 5 | 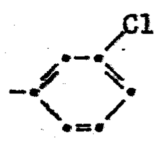 | 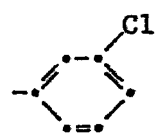 | 358 nm | 25200 |
| 6 | 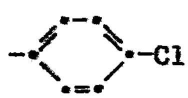 | 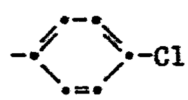 | 361 nm | 28300 |

* DMFA = Dimethylformamid

Beispiel 7: Ausgehend vom 4-Chlor-4'-methyl-pulvinsäurelacton wird gemäss dem im obigen Beispiel 2 angegebenen Verfahren die unsymmetrische Verbindung 2,5-Diketo-3-(4'-methylphenyl)-6-(4"-chlorphenyl)-pyrrolo-[2,3-b]-pyrrol der nachstehenden Formel

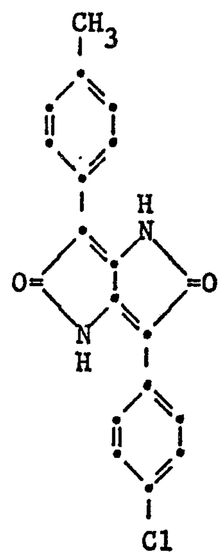

erhalten. Diese Verbindung weist in einer Dimethylformamid-Lösung ein Absorptionsmaximum $\lambda_{max}$ bei 368 nm mit einem $\varepsilon$-Wert von 21500 auf.

Das benötigte 4-Chlor-4'-methyl-pulvinsäure-lacton wird beispielsweise gemäss dem in Beispiel 14 der US-PS 3 944 571 angegebenen Verfahren hergestellt.

Beispiel 8: 0,6 g der Verbindung der Formel I (Bspl. 6, $R_1 = R_2$ = 4-Chlorphenyl) werden mit 67 g Polyvinylchlorid, 33 g Dioctylphthalat, 2 g Dibutylzinndilaurat und 2 g Titandioxid zusammengemischt und auf dem Walzenstuhl während 15 Minuten bei 160°C zu einer dünnen Folie verarbeitet. Die so erzeugte gelbe PVC-Folie ist farbstark, migrations- und lichtbeständig.

Patentansprüche

1. Verfahren zum Färben von hochmolekularem organischem Material in der Masse, dadurch gekennzeichnet, dass ein 2,5-Diketo-pyrrolo[2,3-b]-pyrrol der Formel I

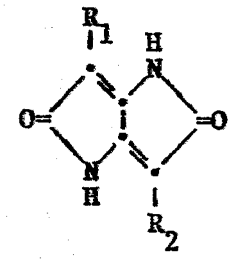

(I)

verwendet wird, wobei $R_1$ und $R_2$ unabhängig voneinander isocyclische oder heterocyclische aromatische Reste bedeuten.

2. Verfahren gemäss Anspruch 1 dadurch gekennzeichnet, dass man Verbindungen der Formel I verwendet, worin $R_1$ und $R_2$ Phenylreste bedeuten.

3. Verfahren gemäss Anspruch 1, dadurch gekennzeichnet, dass man eine Verbindung der Formel I, worin $R_1$ und $R_2$ Reste der Formel II

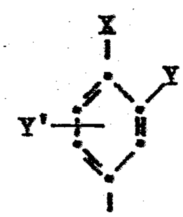

(II)

bedeuten, verwendet, worin X, Y und Y' unabhängig voneinander H- oder Halogenatome, Carbamoyl-, Nitro-, Cyan-, Trifluormethyl-, Alkyl-, Alkoxy- oder Alkylmercaptogruppen mit 1-4 C-Atomen, Alkoxycarbonyl- oder N-Alkylcarbamoylgruppen mit 2-5 C-Atomen, unsubstituierte

oder durch Halogen, Alkyl oder Alkoxy mit 1 - 4 C-Atomen substituierte Phenoxy-, Phenylmercapto-, Phenoxycarbonyl- oder N-Phenylcarbamoylgruppen bedeuten und mindestens einer der Substituenten X, Y und Y' ein Wasserstoffatom bedeutet.

4. Verfahren gemäss Anspruch 1, dadurch gekennzeichnet, dass man Verbindungen der Formel I verwendet, worin $R_1$ und $R_2$ Reste der Formel III

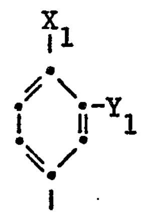

(III)

bedeuten, worin einer der Substituenten $X_1$ und $Y_1$ ein H-, Chlor- oder Bromatom, eine Methyl-, eine Cyangruppe, eine Methoxygruppe, eine Alkoxycarbonyl- oder N-Alkylcarbamoylgruppe mit 2 - 5 C-Atomen oder eine unsubstituierte oder durch Chlor, Methyl oder Methoxy substituierte Phenoxy-, Phenylmercapto- oder N-Phenylcarbamoylgruppe und der andere ein H-Atom bedeuten.

5. Verbindungen der Formel X

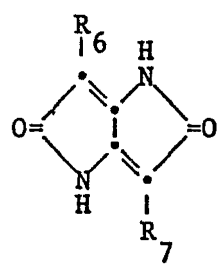

(X),

worin mindestens einer der Reste $R_6$ und $R_7$ einen substituierten Phenylrest bedeutet, ferner $R_6$ und $R_7$ einen carbocyclischen aromatischen oder heterocyclischen aromatischen Rest bedeuten.

6. Verbindungen gemäss Anspruch 5, worin $R_6$ und $R_7$ substituierte Phenylreste bedeuten.

7. Verbindungen gemäss Anspruch 5, worin $R_6$ und $R_7$ identisch sind.

8. Verbindungen gemäss Ansprüchen 5 und 7, worin $R_6$ und $R_7$ Reste der Formel XI

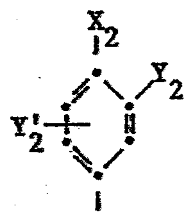

(XI)

bedeuten, worin $X_2$, $Y_2$ und $Y'_2$ unabhängig voneinander Wasserstoff, Halogenatome, Carbamoyl-, Cyan-, Nitro-, Trifluormethyl-, Alkyl-, Alkoxy- oder Alkylmercaptogruppen mit 1 - 4 C-Atomen, Alkoxycarbonyl- oder N-Alkylcarbamoylgruppen mit 2 - 5 C-Atomen, unsubstituierte oder durch Halogen, Alkyl oder Alkoxygruppen mit 1 - 4 C-Atomen substituierte Phenoxy-, Phenylmercapto-, Phenoxycarbonyl- oder N-Phenylcarbamoylgruppen bedeuten und worin mindestens einer der Substituenten $X_2$, $Y_2$ und $Y'_2$ ein H-Atom bedeutet.

9. Verbindungen gemäss Anspruch 5 und 7, worin $R_6$ und $R_7$ Reste der Formel XII

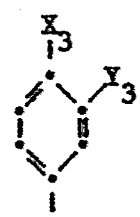

(XII)

bedeuten, worin einer der Reste $X_3$ und $Y_3$ ein Chlor- oder Bromatom, eine Methyl- oder Cyangruppe, eine Methoxygruppe, eine Alkoxycarbonyl- oder N-Alkylcarbamoylgruppe mit 2-5 C-Atomen oder eine unsubstituierte oder durch Chlor, Methyl oder Methoxy substituierte Phenoxy-, Phenylmercapto- oder N-Phenylcarbamoylgruppe und der andere ein H-Atom bedeuten.

10. Verfahren zur Herstellung von 2,5-Diketopyrrolo-[2,3-b]-pyrrolen

der Formel I

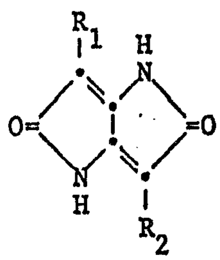

(I),

worin $R_1$ und $R_2$ die in Anspruch 1 angegebene Bedeutung haben, dadurch gekennzeichnet, dass man ein Pulvinsäurederivat der Formel IX oder ein Pulvinsäurelacton der Formel VI

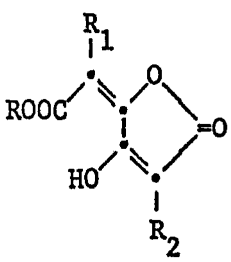

(IX) ,

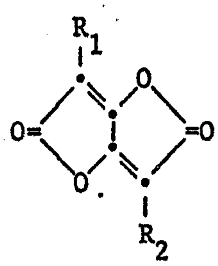

(VI)

in einem organischen Lösungsmittel in Gegenwart eines Ammoniak abgebenden Mittels und eines aliphatischen Alkohols bei erhöhter Temperatur umsetzt, wobei $R_1$ und $R_2$ die im Anspruch 1 angegebene Bedeutung haben und R für $C_1$-$C_8$-Alkyl steht.

11. Verfahren nach Anspruch 10, dadurch gekennzeichnet, dass man als Ammoniak abgebendes Mittel Aethoxyacetamid verwendet.

12. Verfahren nach Anspruch 10, dadurch gekennzeichnet, dass man als aliphatischen Alkohol einen aliphatischen Alkohol mit 1 bis 8 C-Atomen verwendet.

13. In der Masse gefärbtes hochmolekulares Material enthaltend eine Verbindung der Formel I gemäss Anspruch 1.

FO 7.3/RU/we*